# FASCICULE DE BREVET EUROPEEN

(11) **EP 2 931 695 B1**
(45) Date de publication et mention de la délivrance du brevet: **12.04.2017**
(21) Numéro de dépôt: 13824608.7
(22) Date de dépôt: 16.12.2013
(51) Int. Cl.: C07C 67/03, C07C 67/54, C07C 213/06

(54) **PROCEDE DE PRODUCTION D'ACRYLATE D'ALKYLE**
VERFAHREN ZUR HERSTELLUNG VON ALKYLACRYLAT
PROCESS FOR PRODUCING ALKYL ACRYLATE

(30) Priorité: 17.12.2012 FR 1262123
(43) Date de publication de la demande: 21.10.2015
(73) Titulaire: Arkema France, 92700 Colombes (FR)
(72) Inventeur: RIONDEL, Alain, 77178 Saint Pathus (FR); GRAIRE, Coralie, 69290 Grezieu-la-Varenne (FR); ESCH, Marc, 57800 Freyming-Merlebach (FR); LEVRAY, André, 57890 Porcelette (FR)
(74) Mandataire: Bonnel, Claudine
(86) Numéro de dépôt international: PCT/FR2013/053083
(87) Numéro de publication internationale: WO 2014/096648

(56) Documents cités:
- US-A1- 2005 119 500
- DATABASE CA [Online] CHEMICAL ABSTRACTS SERVICE, COLUMBUS, OHIO, US; ENDO, TORU ET AL: "Manufacture of (meth)acrylate esters via purification by distillation", XP002711829, extrait de STN Database accession no. 2005:975871 cité dans la demande -& JP 2005 239564 A (MITSUBISHI RAYON CO., LTD., JAPAN) 8 septembre 2005 (2005-09-08)

## Description

### DOMAINE DE L'INVENTION

La présente invention concerne la production d'acrylate d'alkyle selon un procédé en continu par transestérification.

### ARRIERE-PLAN TECHNIQUE

La production d'ester par transestérification est une réaction largement répandue depuis des décennies dans le domaine des acryliques. Cependant le traitement du brut réactionnel et l'obtention du produit recherché sous forme purifié représente de nombreux enjeux techniques et a fait l'oeuvre de nombreux développements.

La réaction de transestérification met en jeu un acrylate d'alkyle à chaine « courte » dit léger en présence d'un alcool à chaine carbonée « longue » dit lourd en présence généralement de catalyseur et d'inhibiteur de polymérisation, selon la formule (1) générale suivante :

H₂C=CHCOOR₁ + R₂OH ⇆ H₂C=CHCOOR₂ + R₁OH (1)

Afin de déplacer l'équilibre vers la formation d'acrylate d'alkyle à chaine « longue », il est nécessaire d'éliminer l'alcool « léger » produit au cours de la réaction. Cette réaction s'accompagne généralement de réactions secondaires produisant des impuretés qu'il est nécessaire d'éliminer en vue d'obtenir l'acrylate d'alkyle recherché avec une pureté élevée satisfaisant aux exigences techniques liées à son utilisation finale en tant que monomère. L'acrylate d'alkyle ainsi obtenu rentre dans la production de (co)polymères utilisables dans de nombreux domaines d'application.

Par ailleurs, pour des raisons économiques évidentes, les produits valorisables présents dans le mélange brut réactionnel, notamment les réactifs non réagis et le catalyseur, sont dans la mesure du possible recyclés au sein du procédé.

Parallèlement au recyclage des produits valorisables, le produit recherché requiert d'être isolé et purifié. Pour ce faire, de nombreux processus de séparation/purification comportant un ensemble de distillations, d'extractions, et/ou de décantations sont mis en oeuvre.

Par exemple, le document US 6,977,310 divulgue le traitement du brut réactionnel par une première colonne de distillation qui sépare les produits légers et envoie les produits lourds dans une seconde colonne pour séparer le produit recherché du catalyseur et des inhibiteurs de polymérisation.

Le document US 7,268,251 divulgue différents modes de traitement du brut réactionnel comprenant au minimum quatre colonnes de distillation ou de rectification pour purifier le produit recherché, dont un évaporateur pour séparer le catalyseur.

Il s'avère que le procédé décrit dans le document US 7,268,251 est compliqué à mettre en oeuvre à l'échelle industrielle, du fait de l'optimisation des conditions opératoires de la succession de quatre éléments de distillation/rectification, pour obtenir un produit d'une grande pureté et une productivité satisfaisante.

Dans le document JP 2005-239564, il est proposé d'utiliser une colonne de distillation selon la technologie de paroi divisée (« divided wall column »), pour purifier un mélange réactionnel d'esters (méth)acryliques. Cette technologie basée sur la mise en oeuvre d'une seule colonne, présente cependant des inconvénients tels que son coût par rapport à une colonne classique (il faut installer une paroi de séparation), et son inflexibilité vis-à-vis des changements de la nature des flux à traiter.

On connait par ailleurs, le document EP 0968995 décrivant un procédé de production d'acrylates d'alkyle qui divulgue l'utilisation d'une colonne de distillation, où les réactifs et le produit recherché sont récupérés en tête sous forme gazeuse et en pied sous forme liquide respectivement.

En outre, la réaction de transestérification dans ce procédé se produit directement dans la colonne de distillation. Les inconvénients majeurs d'utiliser un catalyseur homogène dans une colonne de distillation sont d'augmenter drastiquement sa consommation due aux reflux des différents effluents, et également en cas de précipitation du catalyseur d'encrasser la colonne de distillation. Dans le cas d'un catalyseur hétérogène, le catalyseur est directement situé dans la colonne de distillation et ne peut donc pas être recyclé de manière continue. De plus, le rendement de la réaction est directement impacté par l'efficacité du catalyseur qui diminue au cours du temps. Le changement de catalyseur représente un coût très élevé puisque le procédé doit être arrêté et la colonne complètement nettoyée entre chaque chargement de catalyseur.

Le traitement du brut réactionnel dérivé de la réaction de transestérification dans le domaine des acryliques a fait l'objet de nombreux développements. Cependant ces procédés ne sont toujours pas satisfaisants. Il existe donc un besoin important de pouvoir disposer d'un procédé de fabrication d'acrylate d'alkyle plus simple, plus performant et ne présentant pas les inconvénients de l'art antérieur à l'échelle industrielle, répondant aux exigences de pureté du produit fabriqué liées à son utilisation finale par exemple en tant que monomère pour la fabrication de latex à faible teneur en composés organiques volatiles.

La demanderesse a trouvé, après diverses expériences et manipulations, un procédé de production d'acrylate d'alkyle comprenant le traitement du mélange brut réactionnel par distillation à l'aide d'une unique colonne simple, ce qui n'a jamais été suggéré dans l'état de la technique.

Selon l'enseignement du document JP 2005-239564, il est nécessaire d'utiliser une colonne selon la technologie de paroi divisée (« divided wall column ») pour obtenir un (méth)acrylate d'alkyle à chaine courte de pureté suffisante, tel que le méthacrylate de butyle. La Demanderesse a trouvé de nombreux inconvénients à cette utilisation, puisqu'il s'agit d'une colonne de taille généralement très supérieure à celle d'une colonne classique, donc de coût plus élevé, et dont la mise en oeuvre est difficile en termes d'optimisation du fonctionnement, de répartition des inhibiteurs de polymérisation introduits, ou de nettoyage en cas de dépôts sur les parois.

De façon surprenante, la Demanderesse a maintenant découvert que l'utilisation d'une colonne simple pour purifier en une seule étape un mélange réactionnel d'acrylate d'alkyle permet d'obtenir un acrylate d'alkyle avec une pureté supérieure à 99,8%.

L'utilisation d'une colonne simple fait appel à une technologie simplifiée, plus flexible et moins onéreuse que celle mise en oeuvre dans le document JP 2005-239564. Il est de plus apparu à la Demanderesse que ce procédé s'applique avantageusement à la production en continu d'acrylates d'alkyle lourds, c'est-à-dire présentant une chaine alkyle comportant plus 4 de atomes de carbone.

Ce procédé permet à la fois une simplification et une amélioration du traitement des effluents par rapport à ceux de l'art antérieur pour atteindre un produit d'une grande pureté et une haute productivité.

Ainsi, de par l'utilisation d'une unique colonne de distillation simple pour le traitement du brut réactionnel, le procédé selon l'invention permet une réduction importante des coûts de purification par rapport à ceux générés dans les procédés actuels.

### RESUME DE L'INVENTION

La présente invention a pour objet un procédé de production en continu d'acrylate d'alkyle par réaction de transestérification entre un acrylate d'alkyle léger et un alcool lourd en présence d'au moins un catalyseur et d'au moins un inhibiteur de polymérisation, représenté par la réaction suivante :

H₂C=CHCOOR₁ + R2OH ⇆ H₂C=CHCOOR₂ + R₁OH

dans laquelle R₁ représente un groupement méthyle ou éthyle, R₂ représente un groupement alkyle linéaire ou ramifié entre 4 et 9 atomes de carbone, pouvant comporter un atome d'azote, la dite réaction étant réalisée dans un réacteur couplé à une colonne de distillation dans laquelle le mélange azéotropique composé d'acrylate d'alkyle léger et d'alcool léger généré par la réaction de transestérification est soutiré en continu, caractérisé en ce que le brut réactionnel comprenant les réactifs non réagis, les produits de réactions formés, le(s) catalyseur(s) et l'/les inhibiteur(s) de polymérisation est envoyé dans une unique colonne de distillation sous pression réduite dans laquelle la distillation permet d'obtenir:
- en tête, un flux de produits légers constitué essentiellement des alcools R₂OH et R₁OH résiduels ainsi que du réactif non réagi H₂C=CHCOOR₁, qui est recyclé au réacteur,
- en partie basse, le produit recherché H₂C=CHCOOR₂ qui est soutiré latéralement en phase gazeuse,
- en pied, un flux de produits de réaction lourds comprenant le(s) catalyseur(s), le(s) inhibiteur(s) de polymérisation et du H₂C=CHCOOR₂, qui est recyclé en partie au réacteur;
et caractérisé en ce que ladite colonne de distillation est une colonne simple renfermant des plateaux de distillation avec ou sans déversoirs, ou renfermant du garnissage de type vrac ou ordonné avec éventuellement des plateaux de recentrage pour la distribution de liquide, pouvant fonctionner à des pressions inférieures à la pression atmosphérique.

Selon un mode de réalisation, le procédé selon l'invention est caractérisé en ce que le catalyseur de transestérification est un titanate d'alkyle, préférentiellement le titane d'éthyle et/ou le titanate de 2-octyle en solution dans le 2-octanol.

Selon une possibilité préférée offerte par l'invention, le procédé est caractérisé en ce que R₂ représente un groupement alkyle linéaire ou ramifié à 8 atomes de carbone, de préférence de 2-octyle.

Selon un mode de réalisation, le procédé selon l'invention est caractérisé en ce que R₁ représente un groupement éthyle.

Selon un mode de réalisation préféré, le procédé selon l'invention est caractérisé en ce que le catalyseur est présent à raison de 5.10⁻⁴ à 5.10⁻² mole par mole de R₂OH de préférence à raison de 10⁻³ à 10⁻² mole par mole de R₂OH.

Selon une possibilité tout particulièrement préférée offerte par l'invention, le procédé selon l'invention est caractérisé en ce qu'il est adapté à la fabrication de l'acrylate de 2-octyle pour laquelle on utilisera comme catalyseur le titane d'éthyle et/ou le titanate de 2-octyle dans le 2-octanol.

De préférence, le procédé selon l'invention est caractérisé en ce que le rapport molaire du composé H₂C=CHCOOR₁ et du composé R₂OH est compris entre 1 et 3, de préférence entre 1,3 et 1,8.

Selon un mode de réalisation tout particulièrement préféré, le procédé selon la présente invention est caractérisé en ce que la réaction de transestérification est effectuée à une pression comprise entre 350 mmHg (soit 0,47 10⁵Pa) et la pression atmosphérique (760 mmHg, soit 10⁵ Pa), et à une température comprise entre 90 °C et 150 °C, de préférence de 100 °C et 130 °C.

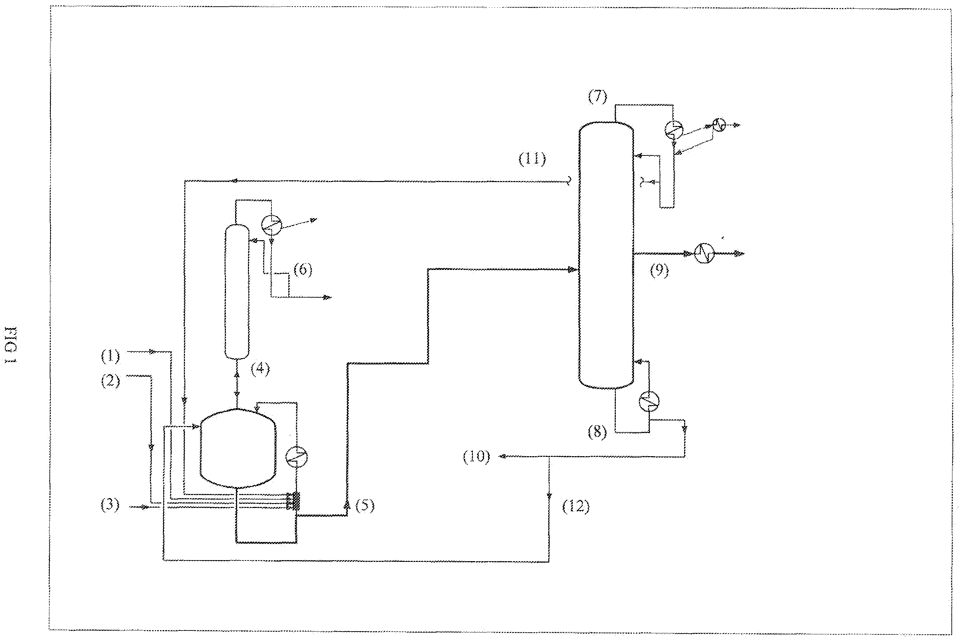

L'invention va maintenant être décrite plus en détails dans la description qui suit, et au regard de la figure unique annexée qui représente de façon schématique une installation pour mettre en oeuvre le procédé de l'invention.

### DESCRIPTION DETAILLEE

En général, le réactif acrylate d'alkyle léger est choisi parmi l'acrylate de méthyle ou l'acrylate d'éthyle, plus préférentiellement l'acrylate d'éthyle.

Selon un mode de réalisation tout particulièrement intéressant du procédé de l'invention, les matières premières sont d'origine naturelle et renouvelable, c'est-à-dire biosourcées. Par exemple, le 2-octanol peut-être obtenu par traitement alcalin de l'acide ricinoléique dérivé de l'huile de ricin.

Plus particulièrement, l'acrylate d'alkyle léger peut-être dérivé d'acide acrylique d'origine renouvelable, pouvant être en particulier obtenu à partir de glycérol, selon un procédé comportant une première étape de déshydratation du glycérol en acroléine suivie d'une étape d'oxydation en phase gaz de l'acroléine ainsi obtenue ; ou obtenu par déshydratation des acides 2-hydroxypropionique (acide lactique) ou 3-hydroxypropionique et de leurs esters.

Le réactif alcool lourd R₂OH est un alcool primaire ou secondaire, présentant une chaine alkyle linéaire ou ramifiée comportant de 4 à 9 atomes de carbone, de préférence de 5 à 9 atomes de carbone, et pouvant comprendre un atome d'azote.

En général, le réactif R₂OH peut-être choisi, sans que cette liste soit limitative, parmi le butan-1-ol, le butan-2-ol, l'iso-butanol, le pentan-1-ol (alcool amylique), le 2,2-méthylpropan-1-ol (l'alcool isoamylique), l'hexan-1-ol, l'alcool benzylique, le 1-octanol, le 2-octanol, le 2-éthylhexanol, le 1-nonanol, le N,N-diméthyl aminoéthanol, le N,N-diéthyl aminoéthanol.

De préférence, l'alcool est choisi parmi le 2-octanol, le 2-éthylhexanol, et le N,N- diméthyl aminoéthanol.

Plus préférentiellement, le réactif R₂OH est le 2-octanol.

Le catalyseur est choisi parmi tous catalyseurs ayant la capacité de catalyser la réaction de transestérification entre un acrylate d'alkyle léger et un alcool léger, par exemples des acides tels que l'acide sulfurique et l'acide p-toluènesulfonique ; des composés basiques tels que des alcoolates, des hydroxydes, des carbonates, des phosphates, des oxydes ou des complexes de métaux alcalins ou alcalino-terreux ; des complexes d'alcoolate de métaux tels que l'alcoolate d'aluminium ou de magnésium ; des composés à base de titane tels que des alcoolates de titane, des phénoxydes de titane ou des titanates d'alkyles ; des composés à base de plomb, de zinc ou d'étain ; des complexes de cuivre, de fer ou de zirconium.

Plus particulièrement, le catalyseur selon le procédé de l'invention est choisi parmi un titanate d'alkyle de formule Ti(OR₁)₄ ou Ti(OR₂)₄ en solution dans l'alcool R₁OH et/ou R₂OH, par exemple une solution de 80 à 90 % de titane d'éthyle dans l'alcool R₂OH ou l'éthanol, et/ou le titanate de R₂OH dans l'alcool R₂OH. Il est entendu que l'alcool utilisé pour mettre en solution le catalyseur est le même que celui utilisé ou généré dans la réaction de transestérification.

Plus préférentiellement, le titanate de 2-octyle en solution dans le 2-octanol, obtenu préalablement par réaction à 100 °C du titanate d'éthyle ou du titanate d'isopropyle avec le 2-octanol est utilisé.

La réaction est effectuée en présence d'un ou plusieurs inhibiteurs de polymérisation qui sont introduits dans le réacteur, à raison de 1000 à 5000 ppm par rapport au mélange brut réactionnel. Comme inhibiteurs de polymérisation utilisables, on peut citer par exemple la phénothiazine, l'hydroquinone, l'éther monométhylique d'hydroquinone, le diterbutyl para-crésol (BHT), le TEMPO (2,2,6,6-tétraméthyl-1-piperidinyloxy), le di-tertiobutylcatéchol, ou les dérivés du TEMPO, tel que le 4-hydroxy (OH)-TEMPO, seuls ou leurs mélanges en toutes proportions. Un ajout supplémentaire d'inhibiteur de polymérisation est généralement effectué au niveau du traitement ultérieur de purification, en particulier au niveau de la colonne de distillation.

L'unique colonne de distillation traitant le brut réactionnel selon le procédé de l'invention est une colonne de distillation simple, par exemple une colonne à garnissage ordonné ou une colonne à plateaux comprenant généralement entre 15 et 20 plateaux théoriques, par exemple 18 plateaux théoriques, dont la pression peut être ajustée au niveau souhaité, éventuellement protégée par une isolation thermique, et éventuellement équipée de dispositifs d'accès pour des opérations de maintenance.

Par colonne de distillation simple, on entend au sens de la présente invention, une colonne renfermant des plateaux de distillation avec ou sans déversoirs, ou renfermant du garnissage de type vrac ou ordonné avec éventuellement des plateaux de recentrage pour la distribution de liquide, pouvant fonctionner à des pressions inférieures à la pression atmosphérique. Cette colonne est équipée d'un système permettant de retirer le produit sous forme vapeur, en position latérale de ladite colonne, et ne comprend pas de paroi de séparation verticale.

Un exemple de fonctionnement du procédé selon l'invention est maintenant décrit en référence à la figure annexée.

L'alcool léger formé (R₁OH) par la réaction de transestérification dans le réacteur entre un acrylate d'alkyle léger (3) et un alcool lourd (1) en présence de catalyseur (2), est entraîné (4) en continu dans une colonne de distillation (CX1) surmontant le réacteur (A) sous forme d'un mélange azéotropique avec l'acrylate d'alkyle léger (3). Le mélange azéotropique peut-être utilisé dans une unité de synthèse d'ester léger (6).

Après réaction, selon un temps de séjour dans le réacteur généralement compris entre 3 et 6 heures, le mélange brut réactionnel (5) contient l'acrylate d'alkyle recherché avec, comme produits légers, l'alcool lourd (R₂OH) et l'acrylate d'alkyle léger n'ayant pas réagi, et comme composés lourds, le catalyseur, le ou les inhibiteurs de polymérisation ainsi que des sous-produits de réaction lourds.

Le mélange brut réactionnel (5) est soumis à un traitement de purification dans une unique colonne de distillation (CX2) afin d'obtenir d'une part l'acrylate d'alkyle (9) recherché pur qui est soutiré latéralement, d'autre part en tête de colonne les alcools résiduels (R₁OH et R₂OH) et l'acrylate d'alkyle léger non réagis (7) destinés à être recyclés (11), et en pied de colonne le catalyseur, inhibiteur de polymérisation, sous-produits de réaction lourds et l'ester lourd (8) destinés à être recyclés en partie (12) au réacteur (A), l'autre partie étant envoyée (10):
- soit vers un évaporateur à film non représenté sur le schéma, pour récupérer un flux de tête contenant l'acrylate recherché et un flux de pied qui est envoyé vers une installation de destruction,
- soit vers une installation de destruction.

La colonne (CX2) est une colonne de distillation simple fonctionnant sous pression réduite, correspondant de préférence à une colonne à garnissage ordonné ou à plateaux comprenant entre 15 et 20 plateaux théoriques, préférentiellement 18 plateaux théoriques opérant sous pression réduite.

De préférence, la colonne de distillation traitant le brut réactionnel fonctionne à une pression comprise entre 20 et 150 mmHg (soit entre 0,027 10⁵ Pa et 0,2 10⁵ Pa), préférentiellement entre 20 et 75 mmHg (soit entre 0,027 10⁵ Pa et 0,1 10⁵ Pa).

De préférence, l'acrylate d'alkyle recherché est soutiré latéralement en phase gazeuse en partie basse de la colonne de distillation, entre les plateaux théoriques 12 et 14 pour une colonne à 18 plateaux théoriques.

Le procédé selon l'invention permet de produire le produit recherché, i.e., acrylate d'alkyle, avec une pureté supérieure à 99,8 %.

Les exemples concrets mais non limitatifs suivants sont donnés afin d'illustrer et de mieux comprendre l'invention.

### PARTIE EXPERIMENTALE

Dans les exemples, les pourcentages sont indiqués en poids sauf indication contraire et les abréviations suivantes ont été utilisées :
AE : acrylate d'éthyle
A2OCT : acrylate de 2-octyle
PTZ : phénothiazine
EMHQ : ester méthylique d'hydroquinone

### Exemple

Dans un réacteur A parfaitement agité, chauffé par un échangeur externe et surmonté d'une colonne à distiller (CX1) de 12 plateaux théoriques, on charge de l'acrylate d'éthyle (3), du 2-octanol (1), un mélange de titanate d'éthyle (2) en solution dans 2-octanol (mélange à 90 % de titanate d'éthyle dans le 2-octanol) avec de l'inhibiteur phénothiazine, dans les proportions massiques 50,1/49,7/0,2.

On met le réacteur en chauffe, sous bullage d'air et dès que la température atteint 125°C sous 640 millibars, on introduit en continu l'AE (3) stabilisé avec 2000 ppm de PTZ, le 2-octanol (1) et le titanate d'éthyle en solution dans le 2-octanol (2), dans des proportions massiques 50,1/49,7/0,2.

En tête de colonne (CX1), on soutire en continu l'azéotrope AE/Ethanol (4) avec une composition massique 40/60. Ce mélange (4) est recyclé sur l'atelier d'ester léger

Le brut réactionnel (5), obtenu par réaction en continu, contient l'A2OCT, l'AE non réagi, le 2-octanol non-réagi et un mélange comprenant le catalyseur avec les inhibiteurs de polymérisation et des dérivés lourds.

Le flux (5) est envoyé en continu vers une unique colonne de distillation (CX2) de 18 plateaux théoriques opérant sous pression réduite et chauffée par un échangeur externe à une température de 140°C.

En tête de colonne (CX2), on introduit un mélange à 2500 ppm de PTZ dans l'AE (non représenté sur la figure).

La colonne CX2 sépare, en tête un mélange (7) AE/2-Octanol/A2OCT de composition 52,4/34,2/4,6 qui est recyclé (11) au réacteur (A), et en pied un mélange (8) comprenant un mélange de produits lourds, inhibiteurs de polymérisation et catalyseur, avec une fraction de A2OCT d'une proportion massique 15,9/84,1, qui est recyclé en partie à la réaction (12), l'autre partie est envoyée vers la section de traitement des lourds (10).

L'acrylate de 2-octyle (9) est obtenu pur par soutirage latéral en phase gazeuse en partie inférieure de la colonne au niveau du plateau N°13. La pureté de l'acrylate de 2-octyle est de 99,89 %.

## Revendications

1. Procédé de production en continu d'acrylate d'alkyle par réaction de transestérification entre un acrylate d'alkyle léger et un alcool lourd en présence d'au moins un catalyseur et d'au moins un inhibiteur de polymérisation, représenté par la réaction suivante :
H₂C=CHCOOR₁ + R₂OH ⇆ H₂C=CHCOOR₂ + R₁OH
dans laquelle R₁ représente un groupement méthyle ou éthyle, R₂ représente un groupement alkyle linéaire ou ramifié entre 4 et 9 atomes de carbone, de préférence entre 5 et 9 atomes de carbone, pouvant comporter un atome d'azote,
la dite réaction étant réalisée dans un réacteur couplé à une colonne de distillation dans laquelle le mélange azéotropique composé d'acrylate d'alkyle léger et d'alcool léger généré par la réaction de transestérification est soutiré,
**caractérisé en ce que** le brut réactionnel comprenant les réactifs non réagis, les produits de réaction formés, le(s) catalyseur(s) et l'/les inhibiteur(s) de polymérisation est envoyé dans une unique colonne de distillation sous pression réduite dans laquelle la distillation permet d'obtenir:
- en tête, un flux de produits légers constitué essentiellement des alcools R₂OH et R₁OH résiduels ainsi que du réactif non réagi H₂C=CHCOOR₁, qui est recyclé au réacteur,
- en partie basse, le produit recherché H₂C=CHCOOR₂ qui est soutiré latéralement en phase gazeuse,
- en pied, un flux de produits de réaction lourds comprenant le(s) catalyseur(s), le(s) inhibiteur(s) de polymérisation et du H₂C=CHCOOR₂ qui, est recyclé en partie au réacteur ;
et **caractérisé en ce que** ladite colonne de distillation est une colonne simple renfermant des plateaux de distillation avec ou sans déversoirs, ou renfermant du garnissage de type vrac ou ordonné avec éventuellement des plateaux de recentrage pour la distribution de liquide, pouvant fonctionner à des pressions inférieures à la pression atmosphérique.

2. Procédé selon la revendication 1 **caractérisé en ce que** l'alcool R₂OH est choisi parmi le 2-octanol, le 2-éthylhexanol, et le N,N- diméthyl aminoéthanol.

3. Procédé selon la revendication 1 ou 2 **caractérisé en ce que** l'alcool est le 2-octanol.

4. Procédé selon l'une quelconque des revendications précédentes **caractérisé en ce que** le catalyseur de transestérification est un titanate d'alkyle de formule Ti(OR₁)₄ ou Ti(OR₂)₄ en solution dans l'alcool R₁OH et/ou R₂OH.

5. Procédé selon l'une quelconque des revendications précédentes **caractérisé en ce que** R₁ représente un groupement éthyle.

6. Procédé selon l'une quelconque des revendications précédentes **caractérisé en ce que** le catalyseur est présent à raison de 5.10⁻⁴ à 5.10⁻² mole par mole de R₂OH, de préférence à raison de 10⁻³ à 10⁻² mole par mole de R₂OH.

7. Procédé selon l'une quelconque des revendications précédentes **caractérisé en ce que** le rapport molaire du composé H₂C=CHCOOR₁ et du composé R₂OH est compris entre 1 et 3, de préférence entre 1,3 et 1,8.

8. Procédé selon l'une quelconque des revendications précédentes **caractérisé en ce que** la réaction de transestérification est effectuée à une pression comprise entre 350 mmHg (0,47 10⁵ Pa) et la pression atmosphérique, et à une température comprise entre 90 °C et 150 °C, de préférence de 100 °C et 130 °C.

9. Procédé selon l'une quelconque des revendications précédentes **caractérisé en ce qu'**il est adapté à la fabrication de l'acrylate de 2-octyle avec comme catalyseur le titane d'éthyle et/ou le titanate de 2-octyle dans le 2-octanol.

## Patentansprüche

1. Verfahren zur kontinuierlichen Herstellung von Alkylacrylat durch eine Umesterungsreaktion zwischen einem leichten Alkylacrylat und einem schweren Alkohol in Gegenwart mindestens eines Katalysators und mindestens eines Polymerisationsinhibitors, dargestellt durch die folgende Reaktion:
H₂C=CHCOOR₁ + R₂OH ⇆ H₂C=CHCOOR₂ + R₁OH
worin R₁ für eine Methyl- oder Ethylgruppe steht, R₂ für eine gerade oder verzweigte Alkylgruppe zwischen 4 und 9 Kohlenstoffatomen, vorzugsweise zwischen 5 und 9 Kohlenstoffatomen, die ein Stickstoffatom tragen können, steht,
wobei die Reaktion in einem Reaktor durchgeführt wird, der mit einer Destillationskolonne gekoppelt ist, in der ein aus leichtem Alkylacrylat und durch die Umesterungsreaktion erzeugtem leichtem Alkohol bestehendes azeotropes Gemisch abgezogen wird,
**dadurch gekennzeichnet, dass** das rohe Reaktionsgemisch, das nicht umgesetzte Reaktanten, die gebildeten Reaktionsprodukte, den/die Katalysator(en) und den/die Polymerisationsinhibitor(en) umfasst, einer einzigen Destillationskolonne unter verringertem Druck zugeführt wird, in der durch Destillation Folgendes erhalten werden kann:
- am Kopf ein Strom leichter Produkte, der im Wesentlichen aus restlichen Alkoholen R₂OH und R₁OH sowie aus dem nicht umgesetzten Reaktanten H₂C=CHCOOR₁ besteht und in den Reaktor zurückgeführt wird,
- im unteren Teil das gewünschte Produkt H₂C=CHCOOR₂, das seitlich in der Gasphase abgezogen wird,
- am Boden ein Strom schwerer Reaktionsprodukte, umfassend den/die Katalysator(en), den/die Polymerisationsinhibitor(en) und H₂C=CHCOOR₂, der zum Teil in den Reaktor zurückgeführt wird,
und **dadurch gekennzeichnet, dass** die Destillationskolonne eine einfache Kolonne ist, die Destillationsböden mit oder ohne Überlaufwehre enthält oder lose oder strukturierte Füllkörper enthält, gegebenenfalls mit Zentrierböden zur Verteilung der Flüssigkeit, die bei Drücken unterhalb von Atmosphärendruck arbeiten kann.

2. Verfahren nach Anspruch 1, **dadurch gekennzeichnet, dass** der Alkohol R₂OH aus 2-Octanol, 2-Ethylhexanol und N,N-Dimethylaminoethanol ausgewählt ist.

3. Verfahren nach Anspruch 1 oder 2, **dadurch gekennzeichnet, dass** der Alkohol 2-Octanol ist.

4. Verfahren nach einem der vorhergehenden Ansprüche, **dadurch gekennzeichnet, dass** der Umesterungskatalysator ein Alkyltitanat der Formel Ti(OR₁)₄ oder Ti(OR₂)₄ in Lösung in dem Alkohol R₁OH und/oder R₂OH ist.

5. Verfahren nach einem der vorhergehenden Ansprüche, **dadurch gekennzeichnet, dass** R₁ für eine Ethylgruppe steht.

6. Verfahren nach einem der vorhergehenden Ansprüche **dadurch gekennzeichnet, dass** der Katalysator zu 5.10⁻⁴ bis 5.10⁻² mol pro mol R₂OH, vorzugsweise zu 10⁻³ bis 10⁻² mol pro mol R₂OH vorliegt.

7. Verfahren nach einem der vorhergehenden Ansprüche, **dadurch gekennzeichnet, dass** das Molverhältnis der Verbindung H₂C=CHCOOR₁ und der Verbindung R₂OH zwischen 1 und 3, vorzugsweise zwischen 1,3 und 1,8 beträgt.

8. Verfahren nach einem der vorhergehenden Ansprüche, **dadurch gekennzeichnet, dass** die Umesterungsreaktion bei einem Druck zwischen 350 mmHg (0,47 10⁵ Pa) und Atmosphärendruck und bei einer Temperatur zwischen 90°C und 150°C, vorzugsweise 100°C und 130°C durchgeführt wird.

9. Verfahren nach einem der vorhergehenden Ansprüche, **dadurch gekennzeichnet, dass** es für die Herstellung von 2-Octylacrylat mit Ethyltitan und/oder 2-Octyltitanat in 2-Octanol als Katalysator ausgelegt ist.

## Claims

1. A process for the continuous production of alkyl acrylate by transesterification reaction between a light alkyl acrylate and a heavy alcohol in the presence of at least one catalyst and at least one polymerization inhibitor, represented by the following reaction:
H₂C=CHCOOR₁ + R₂OH ⇆ H₂C=CHCOOR₂ + R₁OH
in which reaction R₁ represents a methyl or ethyl group, R₂ represents a linear or branched alkyl group of between 4 and 9 carbon atoms, preferably between 5 and 9 carbon atoms, possibly comprising a nitrogen atom,
said reaction being carried out in a reactor coupled to a distillation column from which the azeotropic mixture composed of light alkyl acrylate and light alcohol generated by the transesterification reaction is drawn off,
**characterized in that** the crude reaction product comprising the unreacted reactants, the formed reaction products, the catalyst(s) and the polymerization inhibitor(s) is sent to a single distillation column under reduced pressure, in which the distillation makes it possible to obtain:
- at the top, a stream of light products essentially consisting of the residual alcohols R₂OH and R₁OH and also the unreacted reactant H₂C=CHCOOR₁, which is recycled to the reactor,
- in the bottom portion, the desired product H₂C=CHCOOR₂ which is drawn off laterally in the gas phase,
- at the bottom, a stream of heavy reaction products comprising the catalyst(s), the polymerization inhibitor(s) and H₂C=CHCOOR₂ which is recycled in part to the reactor;
and **characterized in that** the distillation column is a simple column containing distillation plates with or without downcomers, or containing packing of the random or structured type, optionally with recentering plates for the distribution of liquid, able to operate at pressures below atmospheric pressure.

2. The process as claimed in claim 1, **characterized in that** the alcohol R₂OH is chosen from 2-octanol, 2-ethylhexanol and N,N-dimethylaminoethanol.

3. The process as claimed in claim 1 or 2, **characterized in that** the alcohol is 2-octanol.

4. The process as claimed in any one of the preceding claims, **characterized in that** the transesterification catalyst is an alkyl titanate of formula Ti(OR₁)₄ or Ti(OR₂)₄ in solution in the alcohol R₁OH and/or R₂OH.

5. The process as claimed in any one of the preceding claims, **characterized in that** R₁ represents an ethyl group.

6. The process as claimed in any one of the preceding claims, **characterized in that** the catalyst is present in an amount of 5x10⁻⁴ to 5x10⁻² mol per mole of R₂OH, preferably in an amount of 10⁻³ to 10⁻² mol per mole of R₂OH.

7. The process as claimed in any one of the preceding claims, **characterized in that** the molar ratio of the compound H₂C=CHCOOR₁ and of the compound R₂OH is between 1 and 3, preferably between 1.3 and 1.8.

8. The process as claimed in any one of the preceding claims, **characterized in that** the transesterification reaction is carried out at a pressure of between 350 mmHg (0.47 x 10⁵ Pa) and atmospheric pressure, and at a temperature of between 90°C and 150°C, preferably from 100°C to 130°C.

9. The process as claimed in any one of the preceding claims, **characterized in that** it is suitable for the manufacture of 2-octyl acrylate, with ethyl titanate and/or 2-octyl titanate in 2-octanol as the catalyst.
